# EUROPEAN PATENT APPLICATION

(11) **EP 2 444 080 A2**
(43) Date of publication of application: **25.04.2012**
(21) Application number: 12151231.3
(22) Date of filing: 09.02.2007
(51) Int. Cl.: A61K 31/167, A61K 31/58, A61P 11/06, A61K 9/00

(54) **Stable Pharmaceutical Drug Aerosols**

(62) Divisional of application: 07750471.0
(71) Applicant: Schering Corporation, Kenilworth, NJ 07033-0530 (US)
(72) Inventor: Sherwood, Jill K., Edison, NJ New Jersey 08820 (US); Sequeira, Joel A., Venice, FL Florida 34292 (US); Donovan, Brent A., Westfield, NJ New Jersey 07090 (US)
(74) Representative: UEXKÜLL & STOLBERG

(57) **Abstract**

Various aspects of the present invention provide for methods of manufacturing a pharmaceutical drug product, which include storing a container at a temperature greater than ambient conditions for at least about seven days and conducting release testing on the container after storing. Products manufactured by this method have a more consistent fine particle size distribution (FSD) and fine particle fraction (FPF) at ambient conditions and at accelerated stability conditions over the life of the drug product. Advantageously, such products may more reliably and regularly pass testing requirements as required for an approved drug product by regulatory authorities such as the United States Food and Drug Administration (USFDA).

## Description

### Field of Invention

This invention relates to stable pharmaceutical drug products such as stable metered dose inhalers that include an active pharmaceutical agent.

### Background

Pharmaceutical compositions, such as those found in inhalers, may experience stability issues. Stability issues may include degradation of an active pharmaceutical agent (APA) or changes in the delivery of the pharmaceutical composition, such as a change in the particle sizes of an APA emitted from the inhaler. Changes to the particle size distribution of an APA can be particularly problematic since it can alter the effectiveness of the drug product. For instance, an increase in particle size of the drug product may alter the area where the drug product is delivered to a lung. As a result, the effectiveness of the drug product might be diminished.

It was found that the fine particle size distribution (FSD) or fine particle fraction (FPF) for certain active pharmaceutical agents in a drug product changes over a period of time. Any changes to the FSD or FPF may lead questions about the efficacy or safety of the drug product. Additionally, changes to the FSD or FPF are of particular concern with respect to approved drug products since the drug product may fail subsequent stability testing as may be required by a regulatory authority. As such, it would be desirable to provide an inhaler drug product with an active pharmaceutical agent that has a substantially stable fine particle size distribution and can be reliably tested to provide an approved drug product.

### Summary of the Invention

Several embodiments of the present invention provide for methods of manufacturing a pharmaceutical drug product, which include storing a container at a temperature greater than ambient conditions for at least about seven days and conducting release testing on the container after storing. Products manufactured by this method have a more consistent fine particle size distribution (FSD) and fine particle fraction (FPF) at ambient conditions and at accelerated stability conditions over the life of the drug product. Advantageously, such products may more reliably and regularly pass testing requirements as required for an approved drug product by regulatory authorities such as the United States Food and Drug Administration (USFDA). Additionally, such drug products will avoid safety and efficacy concerns with products that may experience a change in FSD or FPF. These methods provide for a drug product that displays substantially the same fine particle distribution and fine particle fraction at ambient conditions over the life of the product, which is typically about two years. The container may include a suspension or solution having at least one active pharmaceutical agent, optionally a propellant and optionally excipients. In several embodiments the APA is at least partially soluble in ethanol.

Various aspects of the present invention provide for a method of manufacturing a pharmaceutical drug product including preparing a container including a suspension or solution including at least one active pharmaceutical agent; and storing the container. Storing the container may be at a temperature greater than ambient conditions for at least about seven days. Alternatively, storing the container can be at a temperature greater than ambient conditions for a period of about 3 days followed by storing the container for about 3 days at ambient conditions and repeating such storing as desired until the at least one APA has a fine particle size distribution and/or a fine particle fraction that remains substantially the same over a period of time, such as sometime between about 2 months and about 2 years when stored at ambient conditions. The container may also include a propellant such as 1,1,1,2-tetrafluoroethane, 1,1,1,2,3,3,3-heptafluoropropane or a combination thereof and optionally excipients. This method may further include conducting release testing on the container after storing.

Another aspect of the invention provides for a pharmaceutical drug product produced by this method. It was found that the particle size distribution of the drug product remained substantially the same over extended periods of time. In several embodiments the at least one active pharmaceutical agent includes mometasone furoate and formoterol fumarate. Useful optional excipients include co-solvents, surfactants and combinations thereof. In various embodiments, ethanol is included as an excipient.

Additional embodiments of the present invention provide for a drug product, which includes a container, such as a metered dose inhaler container or a spray inhaler, such as a nasal spray container- The container includes a pharmaceutical formulation. The formulation may include a propellant and optionally one or more excipients as well as at least one active pharmaceutical agent. In several embodiments the APA has a fine particle size distribution that stays substantially the same over a period of time such as for about 6 months from the date of manufacture when stored at ambient conditions. Various embodiments provide for a drug product having a MDI container which includes ethanol and a propellant, such as 1,1,1,2-tetrafluoroethane, 1,1,1,2,3,3,3-heptafluoropropane. Other embodiments provide for a nasal spray inhaler container which includes one or more excipients. In several particular embodiments, the at least one APA includes a corticosteroid.

Useful excipients include ethanol. Ethanol is a useful excipient since it may act as a co-solvent for APAs that are in need of co-solvent. Ethanol may assist in wetting the APA and reduce surface tension to help dissolve an APA and enable the preparation of a suspension when placed in the presence of a propellant such as 1,1,1,2-tetrafluoroethane, 1,1,1,2,3,3,3-heptafluoropropane. In several embodiments the at least one APA is at least partially soluble in ethanol. At least partially soluble in ethanol includes APAs that are very soluble in ethanol as well as APAs that are partially soluble in ethanol. A drug product that includes ethanol may be considered to be more robust in that it may stay as a better suspension for a longer period of time after shaking.

Ambient conditions typically refer to a temperature range between about 20°C and 25°C-Substantially the same refers to when the fine particle size distribution does not change more than about 20%, preferably not more than about 15%, more preferably not more than 10% and even more preferably not more than about 5%.

Other embodiments provide for a drug product which includes a metered dose inhaler container that includes at least one active pharmaceutical agent, ethanol, a propellant. The at least one active pharmaceutical agent may have a fine particle fraction that stays substantially the same over a period of time for at least about 3 months from the date of manufacture when stored at ambient conditions. The at least one active pharmaceutical agent may include mometasone furoate and optionally formoterol fumarate. Ambient conditions are considered to include temperatures between about 20°C and 25°C. Desirably, the fine particle fraction does not change more than about 20% or more than about 15%, or more than about 10% or more than about 5%. Useful excipients include co-solvents, surfactants, carriers and combinations of two or more thereof. More specifically, useful excipients include lactose, lecithin, oleic acid and combinations of two or more thereof.

Various embodiments of the present invention provide for a method of stabilizing a drug product by storing a drug product for a period of time. The drug product may include a metered dose inhaler container which includes a suspension or solution including at least one active pharmaceutical agent for a period of time of at least about seven days at a temperature above ambient conditions.

Useful storing periods include from about at least 2 weeks to about at least 10 weeks at 40°Clambient RH. More particularly, useful stabilization periods of time include at lease about 2, 3, 4, 5, 6, 7, 8, 9 or about 10 weeks. One particularly useful stability period of time is at least about 6 weeks. Suitable storing or stabilizing times further include a time period of about 2, 3, 4, 5 or 6 weeks. Additional useful storing times include a time period of at least about 2 weeks, of at least about 3 weeks, of at least about 4 weeks, about 5 weeks of at least about 6 weeks. The storing step may occur before release testing is performed on the metered dose inhaler.

Useful storing temperatures include temperatures above ambient conditions. Specifically useful temperatures include temperatures between about 30° C and about 60° C, between about 35°C and about 45°C, about 30°C, about 35°C or about 40°C. In one embodiment the storing conditions are at a temperature about 40°C for a period for at least 2 weeks. In another embodiment the storing conditions are a temperature of about 40°C for about 4, about 5 or about 6 weeks.

In various embodiments, storing may be conducted with a cycling procedure where the product may be stored at a temperature greater than ambient temperature for several days such as about 3 days and then at ambient conditions for several days such as 3 days and repeated as desired until the fine particle size distribution stays substantially the same over a period time such as about 6 months at ambient conditions. Useful temperatures greater than the ambient temperature include from about 30°C to about 60°C, from about 30°C to about 50°C, from about 35°C to about 45°C and at about 40°C.

Useful active pharmaceutical agents include anticholinergics, corticosteroids, long acting beta agonists, phosphodiesterase IV inhibitors and combinations thereof. Such APAs may be useful in the prevention or treatment of a respiratory, inflammatory or obstructive airway disease. Specifically, useful active pharmaceutical agents include mometasone furoate, formoterol fumarate and combinations thereof.

Useful corticosteroids include mometasone furoate; beclomethasone dipropionate; budesonide; fluticasone; dexamethasone; flunisolide; triamcinolone; (22R)-6.alpha.,9.alpha.-difluoro-11.beta.,21-dihydroxy-16.alpha.,17.alpha. -propylmethylenedioxy-4-pregnen-3,20-dione, tipredane, or a pharmaceutically acceptable salt, isomer or hydrate thereof. Useful corticosteroids that are partially soluble in ethanol include beclomethasone dipropionate; flunisolide; triamcinolone; mometasone furoate.

### Brief Description of Drawings

Figure 1 shows the mometasone furoate fine particle distribution for the formoterol fumarate 5 mcg / mometasone furoate 200 mcg MDI through 45 weeks storage (individual ACI data).
Figure 2 shows mometasone furoate ACI deposition profiles for the formoterol fumarate,5 mcg/ mometasone furoate 200 mcg MDI following 2 weeks storage at ambient, 40°Clambient humidity and 40°C/75% RH (mean of n = 5 for each storage condition).
Figure 3 shows formoterol fumarate fine particle dose stabilization data for a full scale manufactured batch, formoterol fumarate 5 mcg/mometasone furoate 200 mcg MDI, following storage at 40°C/75% RH and 40°C/ambient RH.
Figure 4 shows mometasone furoate fine particle dose,stabilization data for a full scale manufactured batch, formoterol fumarate 5 mcg/mometasone furcate 200 mcg MDI, following storage at 40°C/75% RH and 40°C/ambient RH.
Figure 5 shows mometasone furoate fine particle dose stabilization data for formoterol fumarate 5 mcg/mometasone furoate 200 mcg MDI, following storage at 40°C/ambient RH.
Figure 6 shows formoterol fumarate fine particle dose stabilization data for formoterol fumarate 5 mcg/mometasone furoate 200 mcg MDI, following storage at 40°C/ambient RH.

### Detailed Description

During the development of inhalers, it was found that by adding a storing step to the manufacturing process, the particle size distribution did not significantly change over an extended period of time at ambient conditions, both ambient temperature and relative humidity.

Without being bound to any theory, it is believed that drug products having an MDI container that includes some amount of ethanol and a propellant, such as 1,1,1,2-tetrafluoroethane, 1,1,1,2,3,3,3-heptafluoropropane, or a combination thereof and at least one APA that is partially soluble in ethanol may be more susceptible to a change of fine particle size distribution over a period of time. Thus, APAs that are partially soluble in ethanol may be at a higher risk of Ostwald Ripening. Various embodiments provide for a drug product having a MDI container which includes ethanol and a propellant, such as 1,1,1,2-tetrafluoroethane, 1,1,1,2,3,3,3-heptafluoropropane, or a combination thereof and at least one APA that has some solubility in ethanol. APAs that are partially soluble in ethanol include, but are not limited, to triamcinolone, flunisolide, beclomethasone and mometasone furoate.

Thus, various aspects of the present invention provide for a method of manufacturing a pharmaceutical drug product including preparing a container including a suspension or solution including at least one active pharmaceutical agent, a propellant selected from the group consisting of 1,1,1,2-tetrafluoroethane, 1,1,1,2,3,3,3-heptafluoropropane and a combination thereof and optionally excipients; storing said container in at a temperature greater than ambient conditions for at least about seven days.

Useful storing or stabilizing periods include from about at least 2 weeks to about at least 10 weeks at 40°C/ambient RH. More particularly, useful stabilization periods of time include at lease about 2, 3, 4, 5, 6, 7, 8, 9 or about 10 weeks. One particularly useful stability period of time is at least about 6 weeks. Suitable storing or stabilizing times further include a time period of about 2, 3, 4, 5 or 6 weeks. Additional useful storing times include a time period of at least about 2 weeks, of at least about 3 weeks, of at least about 4 weeks, about 5 weeks of at least about 6 weeks. The storing step may occur before release testing is performed on the metered dose inhaler.

Useful storing temperatures include temperatures above ambient conditions. Specifically useful temperatures include temperatures between about 30° C and about 60° C, between about 35°C and about 45°C, about 30°C, about 35°C or about 40°C. In one embodiment the storing conditions are at a temperature about 40°C for a period for at least 2 weeks. In another embodiment the storing conditions are a temperature of about 40°C for about 4, about 5 or about 6 weeks.

In various embodiments, storing may be conducted with a cycling procedure where the product may be stored at a temperature greater than ambient temperature for several days such as about 3 days and then at ambient conditions for several days such as 3 days and repeated as desired until the fine particle size distribution stays substantially the same over a period time such as about 6 months at ambient conditions. Useful temperatures greater than the ambient temperature include from about 30°C to about 60°C, from about 30°C to about 50°C, from about 35°C to about 45°C and at about 40°C.

Useful active pharmaceutical agents include anticholinergics, corticosteroids, long acting beta agonists, phosphodiesterase. IV inhibitors and combinations thereof. Such APAs may be useful in the prevention or treatment of a respiratory, inflammatory or obstructive airway disease. Specifically, useful active pharmaceutical agents include mometasone furoate, formoterol fumarate and combinations thereof.

Useful corticosteroids include mometasone furoate; beclomethasone dipropionate; budesonide; fluticasone; dexamethasone; flunisolide; triamcinolone; (22R)-6.alpha.,9.alpha.-difluoro-11.beta.,21-dihydroxy-16.alpha.,17.alpha. -propylmethylenedioxy-4-pregnen-3,20-dione, tipredane, or a pharmaceutically acceptable salt, isomer or hydrate thereof. Useful corticosteroids that are partially soluble in ethanol include beclomethasone dipropionate; flunisolide; triamcinolone; mometasone furoate.

Useful anticholinergics include (R)-3-[2-hydroxy-2,2-(dithien-2-yl)acetoxy]-1-1[2-(phenyl)ethyl]-1-azoniabicyclo[2.2.2] octane, glycopyrrolate, ipratropium bromide, oxitropium bromide, atropine methyl nitrate, atropine sulfate, ipratropium, belladonna extract, scopolamine, scopolamine methobromide, methscopolamine, homatropine methobromide, hyoscyamine, isopriopramide, orphenadrine, benzalkonium chloride, tiotropium bromide, GSK202405, or a pharmaceutically acceptable salt, isomer or hydrate of any of the above, or a combination of two or more of the above Anticholinergics that are known to be partially soluble in ethanol include but is not limited to, ipratropium and tiotropium.

Useful long acting beta agonists include carmoterol, indacaterol, TA-2005, albuterol, terbutaline, salmeterol, bitolterol, formoterol, fenoterol, metaprotenerol, GSK159802, GSK642444, GSK159797, GSK597901, GSK678077, or a pharmaceutically acceptable salt, isomer or hydrate of any of the above.

Useful phosphodiesterase IV inhibitors cilomilast, roflumilast, tetomilast, 1-[[5-(1(S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester or a pharmaceutically acceptable salt, isomer or hydrate of any of the above.

Mometasone furoate, the active component of NASONEX® is an anti-inflammatory corticosteroid having the chemical name, 9,21-Dichloro-11(beta), 17-dihydroxy-16(alpha)-methylpregna-1,4-diene-3,20-dionel7-(2 Furoate). This component may be present in an amount of about 25 to about 500 micrograms per actuation of the MDI. This product is available from Schering-Plough Corporation, Kenilworth, N.J.

Formoterol fumarate is a selective beta₂-adrenergic bronchodilator. Its chemical name is (±)-2-hydroxy-5-[(1RS)-1-hydroxy-2-[[(1RS)-2-(4-methoxyphenyl)-1-methylethyl]-amino]ethyl] formanilide Fumarate dihydrate. This component may be present in an amount of about 3 to about 50 micrograms per actuation. This product is available commercially from Novartis Corporation, East Hanover, N.J. and Schering-Plough Corporation, Kenilworth, N.J. under the trademark FORADIL®.

Propellant-based pharmaceutical aerosol formulations in the art typically use a mixture of liquid chlorofluorocarbons as the propellant, although many others use a single propellant. As is known in the art, the propellant serves as a vehicle for both the active agents and excipients. Fluorotrichloromethane, dichlorodifluoromethane and dichlorotetrafluoroethane are the most commonly used propellants in aerosol formulations for administration by inhalation. Such chlorofluorocarbons (CFCs), however, have been implicated in the destruction of the ozone layer and their production is being phased out. Non-CFC propellants are said to be less harmful to the ozone than many chlorofluorocarbon propellants. Non-CFC propellants systems must meet several criteria for pressurized metered dose inhalers. They must be non-toxic, stable and non-reactive with the medicament and the other major components in the valve/actuator. One propellant that has been found to be suitable is CF₃CHFCF₃, also known as HFA 227, HFC 227 or 1,1,1,2,3,3,3 heptafluoropropane. Another such propellant for use in metered dose inhalers is CF₃CH₂F, also known as 1,1,1,2-tetrafluoroethane or HFA 134a.

Useful formulations typically may include HFA 227 or HFA 134a, or a combination thereof, in combination with mometasone furoate and optionally, formoterol fumarate, a liquid excipient, and a surfactant. The excipient facilitates the compatibility of the medicament with the propellant and also lowers the discharge pressure to an acceptable range, i.e., about 2.76-5.52×10⁵ newton/meter² absolute (40 to 80 psi), preferably 3.45-4.83×10⁵ newton/meter² absolute (50 to 70 psi). The excipient chosen must be non-reactive with the medicaments, relatively non-toxic, and should have a vapor pressure below about 3.45×10⁵ newton/meter² absolute (50 psi).

Suitable excipients include but are not limited to co-solvents, surfactants, carriers and combinations thereof More specifically, useful excipients include ethanol, oleic acid and combinations thereof.

A surfactant may optionally be added. Surfactants may be any suitable, non-toxic compound that is non-reactive with the medicament and that substantially reduces the surface tension between the medicament, the excipient and the propellant and/or acts as a valve lubricant. Useful surfactants include oleic acid available under the tradenames MEDNIQUE 6322 and EMERSOL 6321 (from Cognis Corp., Cincinnati, Ohio); cetylpyridinium chloride (from Arrow Chemical, Inc. Westwood, N.J.); soya lecithin available under the tradename EPIKURON 200 (from Lucas Meyer Decatur, III.); polyoxyethylene(20)sorbitan monolaurate available under the tradename TWEEN 20 (from ICI Specialty Chemicals, Wilmington, Del.); polyoxyethylene(20)sorbitan monostearate available under the tradename TWEEN 60 (from ICI); polyoxyethylene(20)sorbitan monooleate available under the tradename TWEEN 80 (from ICI); polyoxyethylene (10) stearyl ether available under the tradename BRIJ 76 (from ICI); polyoxyethylene (2) oleyl ether available under the tradename BRIJ 92 (frown ICI); Polyoxyethylene-polyoxypropylene-ethylenediamine block copolymer available under the tradename TETRONIC 150 Rl (from BASF); polyoxypropylene-polyoxyethylene block copolymers available under the tradenames PLURONIC L-92, PLURONIC L-121 end PLURONIC F 68 (from BASF); castor oil ethoxylate available under the tradename ALKASURF CO-40 (from Rhone-Poulenc Mississauga Ontario, Canada); and combinations thereof.

Suitable excipients include "medium chain fatty acids" which refers to chains of alkyl groups terminating in a--COOH group and having 6-12 carbon atoms, preferably 8-10 carbon atoms. The term "short chain fatty acids" refers to chains of alkyl groups terminating in a--COOH group and having 4-8 carbon atoms. The term "alcohol" includes C₁-C₃ alcohols, such as methanol, ethanol and isopropanol.

Other useful excipients include propylene glycol diesters of medium chain fatty acids available under the tradename MIGLYOL 840 (from Huls America, Inc. Piscataway, N.J.); triglyceride esters of medium chain fatty adds available under the tradename MIGLYOL 812 (from Huls); perfluorodimethylcyclobutane available under the tradename VERTREL 245 (from E. I. DuPont de Nemours and Co. Inc. Wilmington, Del.); perfluorocyclobutane available under the tradename OCTAFLUOROCYCLOBUTANE (from PCR Gainsville, Fla.); polyethylene glycol available under the tradename EG 400 (from BASF Parsippany, N.J.); menthol (from Pluess-Stauffer International Stanford, Conn.); propylene glycol monolaurate available under the tradename LAUROGLYCOL (from Gattefosse Elmsford, N.Y.); diethylene glycol monoethylether available under the tradename TRANSCUTOL (from Gattefosse); polyglycolized glyceride of medium chain fatty adds available under the tradename LABRAFAC HYDRO WL 1219 (from Gattefosse); alcohols, such as ethanol, methanol and isopropanol; eucalyptus oil available (from Pluses-Stauffer International); and combinations thereof.

A certain minimum level of ethanol may be used to provide consistent and predictable delivery of the drug from a metered dose dispenser. Suitable minimum levels include about 1 weight percent of the total formulation which results in a marginally acceptable drug delivery. Increased amounts of ethanol generally improve drug delivery characteristics. However, to help prevent drug crystal growth in the formulation, the concentration of ethanol maybe limited.

For formulations containing ethanol, the slurry of bulking agent may be advantageously prepared with an appropriate amount of ethanol. The slurry is subjected to high pressure homogenization prior to adding it to the remainder of the formulation. During manufacture, formoterol and mometasone are typically, initially dispersed with an appropriate amount of HFA 134a and/or HFA 227 and, if applicable or desired ethanol, i.e. such an amount of ethanol to aid dispersion but not so as to result in excessive partial solubilisation of either of the drugs, to prepare a cold concentrate. This dispersion, typically after high shear mixing, is then added to a second appropriate amount of HFA 134a and/or HFA 227 in liquid form, chilled to below its boiling point or range. Thereafter the (homogenized) bulking agent slurry is added. Alternatively the bulking agent slurry may be added to the second appropriate amount of propellant before the drug dispersion is added. If surfactant is used, it is preferably dissolved at ambient temperature in an appropriate amount of ethanol, and it is either added to the drug containing dispersion or the bulking agent slurry.

Products having an aerosol vial equipped with conventional dispensing valves, such as metered dose valves, can be used. Conventional dispensers and aerosol vials may be used to contain a suspension or solution. A glass aerosol vial or a metal, in particular aluminum, vial having an interior surface coated with a polymer, in particular a fluorocarbon polymer may be used. Internal surfaces, in particular such surfaces of components of the valve, or all of the internal surfaces of the dispenser may be coated with a polymer, in particular a fluorocarbon polymer. Suitable fluorocarbon polymers include fluorocarbon polymers, which are made of multiples of one or more of the following monomeric units: tetrafluoroethylene (PTFE), fluorinated ethylene propylene (FEP), perfluoroalkoxyalkane (PFA), ethylene tetrafluoroethylene (ETFE), vinylidenefluoride (PVDF), and chlorinated ethylene tetrafluoroethylene. Polymers, which have a relatively high ratio of fluorine to carbon, such as perfluorocarbon polymers e.g. PTFE, PFA, and FEP.

The valve may be any suitable metering valve with an outlet made from, for example, stainless steel, acetal, nylon or polybutylene terephthalate and with seals made from nitrile or EPDM elastomer.

Useful methods for preparing a container include cold fill process and pressure fill process. For instance, a useful method of preparing includes introducing mometasone furoate anhydrous, a chlorflourocarbon free propellant selected from the group consisting of of 1,1,1,2-tetrafluoroethane, 1,1,1,2,3,3,3-heptafluoropropane and a combination thereof and optionally excipients selected from the group consisting of co-solvents, surfactant and combinations thereof into a vessel that is held under pressure to form a suspension or solution; circulating the suspension or solution from the vessel through a line which includes a filling head; bringing the filling head into communication with the metered dose inhaler container through the valve of said metered dose inhaler container; introducing a quantity of such suspension or solution into the container from the filling head of the line through said valve of the metered dose inhaler container; withdrawing the filling head from the metered dose inhaler container; sealing the metered dose inhaler container.

### Examples

Samples in Tables 1 and 2 are prepared in accordance with the cold fill process. In the cold fill process, chilled propellant HFA 227 is added to a chilled batching vessel and stirred continuously. In a concentrate vessel, a mixture of ethanol and oleic acid is prepared and added to the batching vessel to form a placebo mix. Subsequently, some of the placebo mix is transferred from the batching vessel to a pre-chilled cold concentrate vessel. The active pharmaceutical agent(s) is added to the chilled content in the cold concentrate vessel and mixed. The concentrate is mixed in the cold concentrate vessel and then transferred back into the batching vessel. The resulting formulation is mixed continuously and maintained between about -50°C and about -60°C. The desired quantity of formulation is dispensed into suitable canisters such as FEP internally coated aluminum canisters, which are immediately sealed with metering valves. The units are check-weighed and heat stressed. The units are stored for a minimum of one week, to allow for valve equilibration, 100% function tested for correct valve function and 100% check-weighed. The units are stabilized at 40°Clambient relative humidity for a period of about 4 to about 6 weeks, and 100% check-weighed.

**Table 1 Fine particle distribution stability data following storage at 40°C/75% RH valve down**

| Product Strength | Time Point (weeks) | Formoterol Fumarate (mcg/dose) | Mometasone Furoate (mcg/dose) |
|---|---|---|---|
| | | Mean (% change from initial) | Mean (% change from initial) |
| Formoterol Fumarate (5 mcg) / Mometasone Furoate (50 mcg) delivered dose per actuation | Initial | 6.3 | 60 |
| | 6 | 5.6 (-11.1) | 47 (-21.7) |
| | 13 | 5.5 (-12.7) | 51 (-15.0) |
| | 26 | 5.2 (-17.5) | 50 (-16.7) |
| | 39* | 5.2 (-17.5) | 54 (-10.0) |
| Formoterol Fumarate (5 mcg) / Mometasone Furoate (100 mcg) delivered dose per actuation | Initial | 5.7 | 115 |
| | 13 | 5.0 (-12.3) | 98 (-14.8) |
| | 26 | 4.9 (-14.0) | 97 (-15.7) |
| | 39**** | 5.0 (-12.3) | 101 (-12.2) |
| Formoterol Fumarate (5 mcg) / Mometasone Furoate (200 mcg) delivered dose per actuation | Initial | 5.2 | 213 |
| | 6 | 4.6 (-11.5) | 183 (-14.1) |
| | 13** | 4.5 (-13.5) | 181 (-15.0) |
| | 26*** | 4.5 (-13.5) | 178 (-16.4) |
| | 39**** | 4.4 (-15.4) | 180 (-15.5) |
| * n= 5 at this time point | | | |
| ** n = 9 at this time point | | | |
| *** n = 5 at this time point. | | | |
| **** n =4 at this time point. | | | |

**Table 2 Composition of the Formoterol Fumarate / Mometasone Furoate MDIs**

| Ingredient | Sample | | | | | |
|---|---|---|---|---|---|---|
| | 5/50 mcg Product (mg/metered actuation)* | | 5/100 mcg Product (mg/metered actuation)* | | 5/200 mcg Product (mg/metered actuation)* | |
| | A1 | A2 | B1 | B2 | C1 | C2 |
| Formoterol Fumarate Dihydrate | 0.0067 | 0.0061 | 0.0067 | 0.0061 | 0.0067 | 0.0061 |
| Mometasone Furoate Anhydrous | 0.0670 | 0.0605 | 0.1340 | 0.1210 | 0.2680 | 0.2420 |
| Ethanol/Dehydrated Alcohol | 1.2526 | 1.2526 | 1.2526 | 1.2526 | 1.2526 | 1.2526 |
| Oleic Acid | 0.0035 | 0.0035 | 0.0035 | 0.0035 | 0.0035 | 0.0035 |
| 1,1,1,2,3,3,3-Heptafluoropropane (Propellant 227) | 68.2605 | 68.2676 | 68.1935 | 68.2071 | 68.0595 | 68.0861 |
| Total | 69.5903 | 69.5903 | 69.5903 | 69.5903 | 69.5903 | 69.5903 |
| * This is based on a theoretical valve delivery for a 50 mcl valve. | | | | | | |

As shown in Table 1, the FPD for formoterol fumarate and mometasone furoate show a rAPAd decrease over two weeks at 40°C/ambient RH with a leveling off at subsequent time points during the product stabilization period.

Product stabilization profile is determined by analyzing the FPD on APAs by monitoring the aerodynamic particle size distribution (APSD) using the Anderson Cascade Impaction (ACI). The Andersen sampler (Apparatus 1, US Pharmacopoeia 24 monograph <601>) was used as follows. A filter paper (Whatman 934-AH) was cut to fit the "F" stage, which was incorporated into the stack. The remainder of the stack was assembled, including the USP throat.

Testing consists of priming shots, typically about 4 actuations, are fired through a standard actuator. The valve and valve stem interior are cleaned with water (30 ml) followed by ethanol (30 ml) and dried thoroughly. An unused sample actuator was used to dispense doses while the Andersen sampler was drawing air at 28.3 liter per minute. This was kept sealed into the port of the USP throat with a grommet until 5 seconds after actuation. 6 shots were fired, using the actuation protocol described for uniformity of content. The vacuum source was switched off 30 seconds after the final actuation. The unit was weighed.

The valve stem (including the interior), actuator, throat (including grommet), Stage 0 and inlet cone, plates 0-2, plates 3-5, plates 6-7 and the filter were separately rinsed with an appropriate solvent. The sample diluent was as used for uniformity of content. Each washing is analysed by High Pressure Liquid Chromatography to determine each drug content in micrograms.

The calculation of parameters tabulated is as follows: fine particle fraction (FPF)is determined by the amount of APA analysed from plates 3 to 7 divided by total APA recovered by the rest of the impactor excluding the valve and the actuator from the calculations. The FPF is representative of the drug that might be delivered to the desired area, such as the lung. Fine particle dose is the drug analyzed from plates 3 to 7 and the filter (microgams). Mean metered dose is the total recovery of drug from all diluent samples (micrograms).

During stability testing ofMDI's having a mometasone furoate and formoterol fumarate suspension, a decline in mean fine particle distribution (FPD) was observed following storage at 40°C/75% RH. The data indicated an initial decline in FPD at the 6 week test point, which then remained stable across the subsequent time points as shown in Table 1. No change in the total drug recovered from the cascade impactor was observed over the same period.

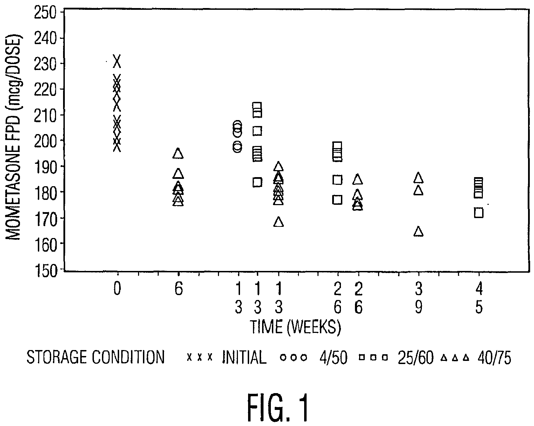

As can be seen in Figure 1, the decrease in FPD was also observed following storage at 25°C/60 RH but to a lesser extent. After 26 weeks of storage, the FPD for mometasone furoate converges to a similar level as seen after 6 weeks when stored at 40°C/75% RH, demonstrating that the FPD stabilizes to the same plateau, but at a different temperature dependent rate.

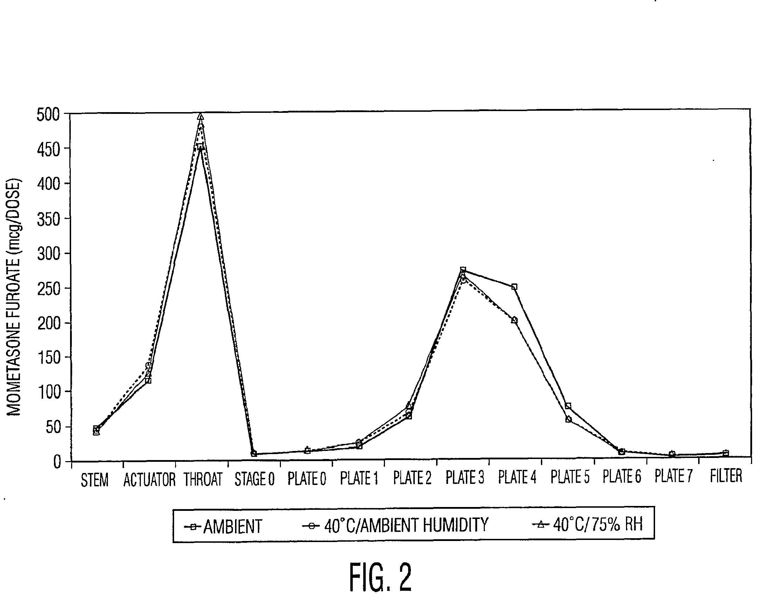

ACI data has provided evidence that the formulation may have undergone a degree of Ostwald ripening. Ostwald Ripening refers to the growth of larger particles from those of smaller size which have a higher solubility than the larger ones. In the process, the larger particles begin to grow at the expense of the smaller particles. This results in an increase of larger fine particle distribution and a corresponding decline in smaller size fine particle distribution. This is consistent with the observed reduction in FPD seen on stability. An example of mometasone furoate ACI deposition profile is shown in Figure 2.

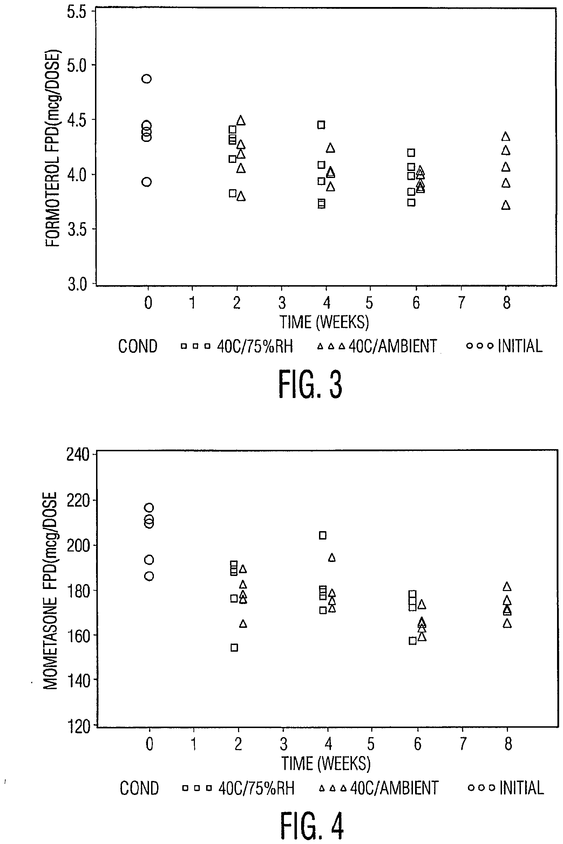

Studies were conducted to determine the effect of temperature, humidity, and time on the stabilization of APSD and FPD of APAs in MDIs. Samples of three different strengths were placed at 40°C/ambient RH and 40°C/75% RH storage conditions. ACI was performed after O. 2, 4 and 6 weeks of storage. Additional ACI was performed after 8 weeks for the units stored at 40°C/ambient RH. FPD data for formoterol fumarate 5mcg and mometasone furoate 200mcg in an MDI are shown in Figure 3 and 4. As shown in Figures 3 and 4, it appears that changes in fine particle size distribution is more prevalent for the mometasone furoate than for the formoterol fumarate.

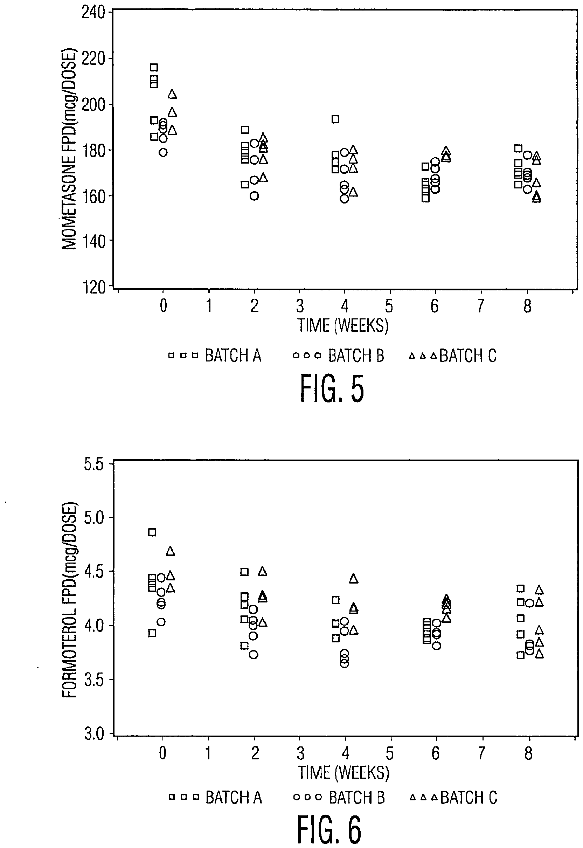

No difference in formoterol fumarate and mometasone furoate FPD performance was observed between the data sets obtained for 40°C/75% RH and 40°C/ambient RH, indicating that stabilization of the FPD performance is not influenced by humidity. All product strengths exhibited comparable trends for FPD performance. Thus, it appears that relative humidity and different amounts/concentrations of APAs have minimal effect on stabilization of the FPD performance. Figures 5 and 6 show that batch to batch differences were non-existent or minimal.

Samples in Table 3 are prepared using a pressure fill process, such as a pressure fill process such as described in US2004/0042973, which is incorporated herein. In a pressure fill process, the excipients such as oleic acid and a portion of ethanol are mixed in a beaker and then added to the chilled batching vessel. The active pharmaceutical agent(s) such as formoterol fumarate and then mometasone furoate are added to the chilled batching vessel. The remaining ethanol is then used to rinse any residual APA that adheres to the charging port into the batching vessel. Sufficient HFA-227 propellant is added to the batching vessel to cover the head of the in dwelling homogenizer. When the homogenizer head is immersed, the homogenizer is turned on and the formulation is mixed at full speed. The remaining HFA-277 propellant is continuously added to the batching vessel while mixing at full speed. Once all of the propellant is added, the speed of the homogenizer is reduced. The formulation is mixed and chilled to -5°C. The requisite quantity of formulation is pressure filled through the metering chamber into the canister such as a FEP internally coated aluminum canisters, which had been previously sealed by vacuum crimping to the metering valve. The units are check-weighed and heat stressed in a water bath. The units are stored for a minimum of one week to allow for valve equilibration. The units are stabilized at 40°C/ambient RH for a period of 6 weeks.

**Table 3 Compositions with Formoterol Fumarate / Mometasone Furoate 5 mcg/25 mcg pMDI**

| Ingredient | Sample (Theoretical Quantity in mg/g) | | | | | |
|---|---|---|---|---|---|---|
| | A | B | C | D | E | F |
| Formoterol Fumarate Dihydrate | 0.0914 | 0.0914 | 0.0914 | 0.0914 | 0.1827 | 0.1827 |
| Mometasone Furoate Anhydrous | 0.4562 | 0.4562 | 0.4562 | 0.4562 | 0.9125 | 0.9125 |
| Ethanol/Dehydrated Alcohol | 18.0000 | 14.0000 | 14.0000 | 10.0000 | 18.0000 | 16.0000 |
| Oleic Acid | 0.0500 | 0.0500 | 0.0000 | 0.0500 | 0.0500 | 0.0500 |
| 1,1,1,2,3,3,3-Heptafluoropropane (Propellant 227) | 981.4024 | 985.4024 | 985.4524 | 989.4024 | 980.8548 | 982.8548 |
| Total | 1000,0000 | 1000.0000 | 1000.0000 | 1000.0000 | 1000.0000 | 1000.0000 |
| Metering Valve (mcl) | 50 | 50 | 50 | 50 | 27 | 27 |
| Canister Size (mL) | 15* | 15* | 15* | 15* | 10 | 10 |

**Table 4 Fine Particle Fraction* Results of Samples D-I Stored at 40°C/75% Humidity**

| Sample | Time at 40/75 (mo) | Beginning of Canister | | | | End of Canister | | | |
|---|---|---|---|---|---|---|---|---|---|
| | | Ave MF FPF (%) | % change from initial | Ave. FF FPF (%) | % change from initial | Ave MF FPF (%) | % change from initial | Ave. FF FPF (%) | % change from initial |
| D | Initial | 37.8% | N/A | 38.0% | N/A | 41.5% | N/A | 40.6% | N/A |
| | 1 mo | 36.8% | -2.6% | 36.4% | -4.2% | 36.9% | -11.1% | 36.4% | -10.3% |
| | 3 mo | 38.5% | 1.9% | 37.4% | -1-6% | 36.6% | -11.8% | 37.1% | -8.6% |
| E | Initial | 46.6% | N/A | 43.2% | N/A | 47.0% | N/A | 43.2% | N/A |
| | 1 mo | 44.7% | -4.1% | 42.9% | -0.7% | 42.4% | -9.8% | 41.1% | -4.9% |
| | 3 mo | 42.8% | -8.2% | 41.1% | -4.9% | 40.9% | -13.0% | 40.1 % | -7.2% |
| F | Initial | 46.1% | N/A | 43.2% | N/A | 46.1% | N/A | 43.3% | N/A |
| | 1 mo | 42.7% | -7.4% | 40.3% | -6.7% | 44.3% | -3.9% | 42.4% | -2.1% |
| | 3 mo | 43.4% | -5.9% | 41.7% | -3.5% | 42.7% | -7.4% | 41.8% | -3.5% |
| G | Initial | 52.8% | N/A | 48.7% | N/A | 53.2% | N/A | 47.9% | N/A |
| | 1 mo | 51.8% | -1.9% | 47.5% | -2.5% | 49.9% | -6.2% | 47.1% | -1.7% |
| | 3 mo | 52.4% | -0.8% | 47.8% | -1.8% | 50.3% | -5.5% | 49.0% | 2.3% |
| H | Initial | 49.0% | N/A | 50.4% | N/A | 48.8% | N/A | 49.8% | N/A |
| | 1 mo | 49.2% | 0.4% | 50.2% | -0.4% | 49.3% | 1.0% | 50.6% | 1.6% |
| | 3 mo | 51.5% | 5.1% | 52.1% | 3.4% | 50.8% | 4.1% | 50.8% | 2.0% |
| I | Initial | 53.3% | N/A | 55.4% | N/A | 53.0% | N/A | 54.9% | N/A |
| | 1 mo | 50.7% | -4.9% | 51.6% | -6.9% | 52.0% | -1.9% | 54.3% | -1.1% |
| | 3 mo | 52.4% | -1.7% | 53.3% | -3.8% | 52.5% | -0.9% | 51.3% | -6.6% |

| | | | | | | | | | |
|---|---|---|---|---|---|---|---|---|---|
| *FPF is calculated without actuator and valve stem deposition. | | | | | | | | | |

**Table 5 Fine Particle Fraction* Results of samples D-I Stored at 25°C/60% Humidity**

| Sample | Time at 25C/60 (mo) | Beginning of Canister | | | | End of Canister | | | |
|---|---|---|---|---|---|---|---|---|---|
| | | Ave MF FPF (%) | % change from initial | Ave. FF FPF (%) | % change from initial | Ave MF FPF (%) | % change from initial | Ave. FF FPF (%) | % change from initial |
| D | Initial | 37.8% | N/A | 38.0% | N/A | 41.5% | N/A | 40.6% | N/A |
| | 3 mo | 38.3% | 1.3% | 37.5% | -1.3% | | -100.0% | | -100.0% |
| E | Initial | 46.6% | N/A | 43.2% | N/A | 47.0% | N/A | 43.2% | N/A |
| | 3 mo | 44.6% | -4.3% | 41.7% | -3.5% | | -100.0% | | -100.0% |
| F | Initial | 46.1% | N/A | 43.2% | N/A | 46.1% | N/A | 43.3% | N/A |
| | 3 mo | 45.7% | -0.9% | 43.1% | -0.2% | | -100.0% | | -100.0% |
| G | Initial | 52.8% | N/A | 48.7% | N/A | 53.2% | N/A | 47.9% | N/A |
| | 3 mo | 53.6% | 1.5% | 48.6% | -0.2% | | -100.0% | | -100.0% |
| H | Initial | 49.0% | N/A | 50.4% | N/A | 48.8% | N/A | 49.8% | N/A |
| | 3 mo | 49.8% | 1.6% | 51.6% | 2.4% | | -100.0% | | -100.0% |
| I | Initial | 53.3% | N/A | 55.4% | N/A | 53.0% | N/A | 54.9% | N/A |
| | 3 mo | 52.6% | -1.3% | 54.6% | -1.4% | | -100.0% | | -100.0% |
| *FPF is calculated without actuator and valve stem deposition. | | | | | | | | | |

The samples of Table 3 were tested in accordance with the samples in Table 2. As shown in Tables 3 and 4, the FPF for formoterol fumarate and mometasone furoate stays substantially the same over the period of time tested at increased stability conditions.

The foregoing descriptions of various embodiments of the invention are representative of various aspects of the invention, and are not intended to be exhaustive or limiting to the precise forms disclosed. Many modifications and variations may occur to those having skill in the art. It is intended that the scope of the invention shall be fully defined solely by the appended claims.

The following represent further embodiments of the present invention:
1. A method of manufacturing a pharmaceutical drug product comprising:
   a) storing a container at a temperature greater than ambient conditions for at least about seven days, wherein said container comprises a suspension or solution comprising at least one active pharmaceutical agent, a propellant selected from the group consisting of 1,1,1,2-tetrafluoroethane, 1,1,1,2,3,3,3-heptafluoropropane and a combination thereof and optionally excipients; and
   b) conducting release testing on said container after said storing.
2. The method of embodiment 1, wherein said active pharmaceutical agent is partially soluble in ethanol.
3. A pharmaceutical drug product produced by the method of embodiment 1.
4. The method of embodiment 1, wherein said excipients are selected from the group consisting of co-solvents, surfactants, carriers and combinations of two or more thereof.
5. The method of embodiment 1, wherein said excipients are selected from the group consisting of ethanol, oleic acid and combinations of two or more thereof.
6. The method according to embodiment 1, wherein storing is for a period of time of at least about 2 weeks.
7. The method according to embodiment 1, wherein storing is for a period of time of at least about 3 weeks.
8. The method according to embodiment 1, wherein storing is for a period of time of at least about 4 weeks.
9. The method according to embodiment 1, wherein storing is for a period of time of at least about 5 weeks.
10. The method according to embodiment 1, wherein storing is for a period of time of at least about 6 weeks.
11. The method according to embodiment 1, wherein said temperature is between about 30° C and about 60° C.
12. The method of embodiment 1, wherein said temperature is between about 35°C and about 45°C.
13. The method of embodiment 1, wherein said temperature is about 40°C.
14. The method of embodiment 1, wherein said temperature is about 40°C and storing is for a period for at least 2 weeks.
15. The method of embodiment 1, wherein said at least one active pharmaceutical agent comprises mometasone furoate.
16. The method of embodiment 1, wherein said at least one active pharmaceutical agent comprises mometasone furoate and formoterol fumarate.
17. The formulation of embodiment 1, wherein said at least one active pharmaceutical agent comprises a corticosteroid selected from the group consisting of mometasone furoate; beclomethasone dipropionate; budesonide; fluticasone; dexamethasone; flunisolide; triamcinolone; (22R)-6.alpha.,9.alpha.-difluoro-11.beta.,21-dihydroxy-16.alpha.,17.alpha. - propylmethylenedioxy-4-pregnen-3,20-dione, tipredane, or a pharmaceutically acceptable salt, isomer or hydrate thereof
18. The method according to embodiment 1, wherein the at least one active pharmaceutical agent is selected from the group consisting of an anticholinergic, a corticosteroid, a long acting beta agonist, phosphodiesterase IV inhibitor and combinations of two or more thereof.
19. The method of embodiment 1, wherein said container is manufactured by a method comprising:
   a) introducing at least one active pharamaceutical agent, a chlorflourocarbon free propellant selected from the group consisting of 1,1,1,2-tetrafluoroethane, 1,1,1,2,3,3,3-heptafluoropropane and a combination thereof and optionally excipients selected from the group consisting of co-solvents, surfactant and combinations of two or more thereof, into a vessel that is held under pressure to form a suspension or solution;
   b) circulating said suspension or solution from the vessel through a line which includes a filling head;
   c) bringing said filling head into communication with said metered dose inhaler container through said valve of said metered dose inhaler container;
   d) introducing a quantity of such suspension or solution into the container from the filling head of the line through said valve of said metered dose inhaler container;
   e) withdrawing said filling head from said metered dose inhaler container;
   f) sealing said metered dose inhaler container.
20. The method of embodiment 5, wherein said container is manufactured by a method comprising:
   a) mixing oleic acid and a portion of ethanol in a beaker and then adding to a chilled batching vessel;
   b) adding the at least one active pharmaceutical agent to the chilled batching vessel;
   c) rinsing any residual active pharmaceutical agent in the charging port into the batching vessel with ethanol;
   d) mixing the ingredients until all of the desired amount of propellant to the batching vessel to cover the head of the in dwelling homogenizer;
   e) chilling the ingredients to the desired temperature; and
   f) pressure filling the desired amount of mixed ingredients into the containers.
21. A drug product comprising a metered dose inhaler container comprising a propellant selected from the group consisting of 1,1,1,2-tetrafluoroethane, 1,1,1,2,3,3,3-heptafluoropropane, and a combination thereof, and at least one active pharmaceutical agent having a fine particle size distribution that stays substantially the same over a period of time for about 6 months from the date of manufacture when stored at ambient conditions; wherein said at least one active pharmaceutical agent comprises a corticosteroid.
22. The product of embodiment 21, wherein said active pharmaceutical agent is partially soluble in ethanol.
23. The product of embodiment 21, further comprising excipients selected from the group consisting of co-solvents, surfactants, carriers and combinations of two or more thereof.
24. The product of embodiment 21, further comprising excipients selected from the group consisting of lactose, lecithin, ethanol, oleic acid and combinations of two or more thereof.
25. The product of embodiment 21, wherein said corticosteroid is selected from the group consisting of mometasone furoate; beclomethasone dipropionate; budesonide; fluticasone; dexamethasone; flunisolide; triamcinolone; (22R)-6.alpha.,9.alpha.-difluoro-11.beta.,21-dihydroxy-16.alpha.,17-alpha. -propylmethylenedioxy-4-pregnen-3,20-dione, tipredane, or a pharmaceutically acceptable salt, isomer or hydrate thereof.
26. The product of embodiment 21, wherein said at least one active pharmaceutical agent comprises mometasone furoate.
27. The product of embodiment 21, wherein said at least one active pharmaceutical agent comprises mometasone furoate and formoterol fumarate.
28. The product of embodiment 21, wherein said ambient conditions comprise a temperature between about 20°C and 25°C.
29. The product of embodiment 21, wherein the fine particle size distribution does not change more than about 20%.
30. The product of embodiment 21, wherein the fine particle size distribution does not change more than about 10%.
31. A drug product comprising a metered dose inhaler container comprising at least one active pharmaceutical agent, ethanol, a propellant selected from the group consisting of 1,1,1,2-tetrafluoroethane, 1,1,1,2,3,3,3-heptafluoropropane, and a combination thereof; wherein said at least one active pharmaceutical agent has a fine particle fraction that stays substantially the same over a period of time for at least about 3 months from the date of manufacture when stored at ambient conditions; wherein said at least one active pharmaceutical agent comprises mometasone furoate.
32. The product of embodiment 31, wherein said at least one active pharmaceutical agent further comprises formoterol fumarate.
33. The product of embodiment 31, wherein said ambient conditions comprise a temperature between about 20°C and 25°C.
34. The product of embodiment 31, wherein the fine particle fraction does not change more than about 20%.
35. The product of embodiment 31, wherein the fine particle fraction does not change more than about 15%.
36. The product of embodiment 31, wherein the fine particle fraction does not change more than about 10%.
37. The product of embodiment 31, wherein the fine particle fraction does not change more than about 5%.
38. The product of embodiment 31, wherein the at least one active pharmaceutically active agent is selected from the group consisting of an anticholinergic, a corticosteroid, a long acting beta agonist, phosphodiesterase IV inhibitor and combinations of two or more thereof.
39. The product of embodiment 31, further comprising excipients selected from the group consisting of co-solvents, surfactants, carriers and combinations of two or more thereof.
40. The product of embodiment 31, further comprising excipients selected from the group consisting of lactose, lecithin, oleic acid and combinations of two or more thereof.

## Claims

1. A drug product produced by a method of manufacturing comprising:
a) storing a container at a temperature greater than ambient conditions for at least about seven days, wherein said container comprises a suspension or solution comprising at least one active pharmaceutical agent, a propellant selected from the group consisting of 1,1,1,2-tetrafluoroethane, 1,1,1,2,3,3,3-heptafluoropropane and a combination thereof and optionally excipients; and
b) conducting release testing on said container after said storing.

2. The product of claim 1, wherein said active pharmaceutical agent is partially soluble in ethanol.

3. The product according to claim 1, wherein storing is for a period of time of at least about 2 weeks, at least about 3 weeks, at least about 4 weeks, at least about 5 weeks or at least about 6 weeks.

4. The product according to claim 1, wherein said temperature is between about 30°C and about 60°C.

5. The product of claim 1, wherein said at least one active pharmaceutical agent comprises mometasone furoate and formoterol fumarate.

6. The product of claim 1, wherein said at least one active pharmaceutical agent comprises a corticosteroid selected from the group consisting of mometasone furoate; beclomethasone dipropionate; budesonide; fluticasone; dexamethasone; flunisolide; triamcinolone; (22R)-6.alpha., 9.alpha.-difluoro-11.beta.,21-dihydroxy-16.alpha.,17.alpha. -propylmethylenedioxy-4-pregnen-3,20-dione, tipredane, or a pharmaceutically acceptable salt, isomer or hydrate thereof.

7. A drug product comprising a metered dose inhaler container comprising a propellant selected from the group consisting of 1,1,1,2-tetrafluoroethane, 1,1,1,2,3,3,3-heptafluoropropane, and a combination thereof, and at least one active pharmaceutical agent having a fine particle size distribution that stays substantially the same over a period of time for about 6 months from the date of manufacture when stored at ambient conditions; wherein said at least one active pharmaceutical agent comprises a corticosteroid.

8. The product of claim 7, wherein said corticosteroid is selected from the group consisting of mometasone furoate; beclomethasone dipropionate; budesonide; fluticasone; dexamethasone; flunisolide; triamcinolone; (22R)-6.alpha., 9.alpha.-difluoro-11.beta.,21-dihydroxy-16.alpha.,17.alpha. -propylmethylenedioxy-4-pregnen-3,20-dione, tipredane, or a pharmaceutically acceptable salt, isomer or hydrate thereof.

9. The product of claim 7, wherein said at least one active pharmaceutical agent comprises mometasone furoate.

10. The product of claim 7, wherein the fine particle size distribution does not change more than about 20%.

11. A drug product comprising a metered dose inhaler container comprising at least one active pharmaceutical agent, ethanol, a propellant selected from the group consisting of 1,1,1,2-tetrafluoroethane, 1,1,1,2,3,3,3-heptafluoropropane, and a combination thereof; wherein said at least one active pharmaceutical agent has a fine particle fraction that stays substantially the same over a period of time for at least about 3 months from the date of manufacture when stored at ambient conditions; wherein said at least one active pharmaceutical agent comprises mometasone furoate.

12. A method of manufacturing a pharmaceutical drug product comprising:
a) storing a container at a temperature greater than ambient conditions for at least about seven days, wherein said container comprises a suspension or solution comprising at least one active pharmaceutical agent, a propellant selected from the group consisting of 1,1,1,2-tetrafluoroethane, 1,1,1,2,3,3,3-heptafluoropropane and a combination thereof and optionally excipients; and
b) conducting release testing on said container after said storing.

13. The method of claim 12, wherein said active pharmaceutical agent is partially soluble in ethanol.

14. The method according to claim 12, wherein said temperature is between about 30°C and about 60°C.

15. The method of claim 12, wherein said at least one active pharmaceutical agent comprises mometasone furoate and formoterol fumarate.
